(19) Europäisches Patentamt
European Patent Office
Office européen des brevets

(11) Veröffentlichungsnummer: **0 008 322**
**B1**

# (12) EUROPÄISCHE PATENTSCHRIFT

(45) Veröffentlichungstag der Patentschrift: **05.08.81**

(51) Int. Cl.³: **C 07 C 37/00,** C 07 C 39/08, B 01 J 23/96, B 01 J 23/56

(21) Anmeldenummer: **79101671.0**

(22) Anmeldetag: **31.05.79**

## (54) Verfahren zur Herstellung von Resorcinen aus Delta-Ketocarbonsäureestern und Verfahren zur Regenerierung des verwendeten Katalysators.

(30) Priorität: **08.06.78 DE 2825073**

(43) Veröffentlichungstag der Anmeldung: **05.03.80 Patentblatt 80/5**

(45) Bekanntmachung des Hinweises auf die Patenterteilung: **05.08.81 Patentblatt 81/31**

(84) Benannte Vertragsstaaten: **BE CH DE FR GB IT NL**

(56) Entgegenhaltungen:
**DE-A-2 245 607**
**DE-A-2 259 607**
**DE-A-2 412 371**
**DE-A-2 437 983**
**DE-A-2 450 086**
**DE-A-2 533 920**
**DE-A-2 749 437**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Müller, Werner Heinrich, Dr., Taunusblick 5, D-6239 Eppstein/Taunus (DE)**
Erfinder: **Mack, Karl Ernst, Dr., Kirchplatz 14, D-6233 Kelkheim (Taunus) (DE)**
Erfinder: **Hey, Hansjörg, Dr., Auwald 8, D-6238 Hofheim am Taunus (DE)**

**EP 0 008 322 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Resorcinen aus Delta-Ketocarbonsäureestern und Verfahren zur Regenerierung des verwendeten Katalysators.

Die Erfindung betrifft ein Verfahren zur Herstellung von Resorcinen durch katalytische Umsetzung von δ-Ketocarbonsäureestern in der Gasphase in Gegenwart von Wasserstoff.

Resorcine finden als Kunstharzkomponenten in der Gummi- und Holzindustrie, als Kupplungskomponenten in der Diazotypie und als Antiseptika Verwendung.

Es ist bekannt, dass durch Cyclisierung von δ-Ketocarbonsäureestern in der Flüssigphase mittels starker Basen Cyclohexan-1.3-dione hergestellt und anschliessend durch Dehydrierung in der Flüssigphase in Resorcine übergeführt werden können (DE-OS 25 33 920). Es ist weiterhin bekannt, dass Resorcine durch katalytische Dehydrocyclisierung von δ-Ketocarbonsäuren oder deren Lactonen (DE-OS 24 50 086) oder von δ-Ketocarbonsäureestern (DE-OS 24 12 371) in der Gasphase hergestellt werden können.

Die Wirtschaftlichkeit eines grosstechnischen Verfahrens wird entscheidend verbessert, wenn es einstufig, in der Gasphase und ohne Anfall wertloser, umweltbelastender Nebenprodukte, wie beispielsweise anorganische Salze, durchführbar ist. Weiter sind wichtig: hohe Selektivität der Reaktion, hoher Umsatz, und – falls Edelmetallkatalysatoren verwendet werden – eine hohe Ausbeute relativ zur Edelmetallmenge, eine hohe Lebensdauer bzw. eine gute Regenerierbarkeit.

Das genannte Flüssigphaseverfahren zur Herstellung von Resorcinen, bei dem Cyclohexan-1.3-dione als Zwischenprodukte auftreten, hat den Nachteil, zweistufig und unter Bildung stöchiometrischer Mengen, anorganische Salze abzulaufen. Die beiden genannten Gasphaseverfahren zeigen relativ geringe Umsätze und relativ kleine Ausbeuten relativ zur Edelmetallmenge.

Es wurde nun ein Verfahren zur Herstellung von Resorcinen der allgemeinen Formel

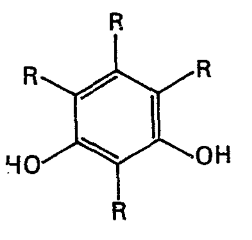

in der die einzelnen Reste R gleich oder verschieden und unabhängig voneinander Wasserstoff, Alkyl, Cycloalkyl, Aryl, Alkoxy oder Carbalkoxy mit einer Gesamtzahl von bis zu 12 Kohlenstoffatomen sein können, durch Umsetzung von δ-Ketocarbonsäureestern der allgemeinen Formel

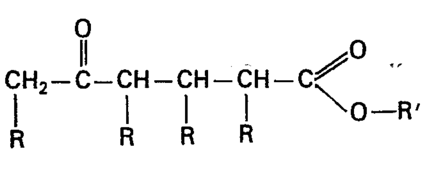

wobei R obige Bedeutung hat und R' ein Alkyl-, Cycloalkyl- oder Aryl-Rest mit bis zu 8 Kohlenstoffatomen ist, in der Gasphase bei Temperaturen von 250 bis 500°C in Gegenwart von Wasserstoff an einem Katalysator gefunden, das dadurch gekennzeichnet ist, dass der Katalysator aus 2 Komponenten besteht, wobei die erste Komponente aus einem oder mehreren Trägermaterialien besteht, auf die jeweils mindestens eine Verbindung eines Metalls der Gruppe VIII und/oder mindestens eine Verbindung eines Metalls der Gruppe I B aufgebracht worden ist, und wobei die zweite Komponente aus einem oder mehreren Trägermaterialien besteht, auf die jeweils mindestens eine Verbindung eines Metalls der Gruppe II A und/oder IV A und/oder III B und/oder IV B des Periodensystems aufgebracht worden ist.

Der Katalysator besteht also aus 2 verschiedenen Komponenten, wobei die erste Komponente mindestens ein Element aus der folgenden Klasse, die als Klasse I bezeichnet ist, enthält:

Eisen, Cobalt, Nickel, Kupfer, Ruthenium, Rhodium, Palladium, Silber, Osmium, Iridium, Platin, Gold, und wobei die zweite Komponente mindestens ein Element aus der folgenden Klasse enthält, die als Klasse II bezeichnet sei:

Beryllium, Magnesium, Calcium, Strontium, Barium, Germanium, Zinn, Blei, Scandium, Yttrium, Lanthan, Lanthaniden, Titan, Zirkon, Hafnium, Thorium, Uran.

Enthält die erste Komponente lediglich ein Element aus der Klasse I, so kann dieses auf ein einziges Trägermaterial oder auch auf mehrere verschiedene Trägermaterialien aufgebracht werden. Enthält die erste Komponente mehrere Elemente aus der Klasse I, so kann man alle diese Elemente auf ein einziges Trägermaterial aufbringen, man kann aber auch mehrere verschiedene Trägermaterialien verwenden und auf jedes von diesen ein oder mehrere der betreffenden Elemente aufbringen.

Analoges gilt für das Aufbringen eines oder mehrerer Elemente der Klasse II auf Trägermaterial zur Herstellung der zweiten Komponente. In der zweiten Komponente kann man dabei das gleiche oder mehrere gleiche Trägermaterialien wie in der ersten Komponente verwenden, man kann aber auch ein anderes oder mehrere andere Trägermaterialien einsetzen.

Es ist also wesentlich, dass die beiden Katalysatorkomponenten separat hergestellt, und erst nach ihrer Herstellung zusammengebracht werden, um den Katalysator des erfindungsgemässen Verfahrens zu bilden. Dieser Katalysator, der sich aus zwei separat hergestellten Komponenten zusammensetzt, hat gegenüber Katalysatoren, die Elemente aus der Klasse I und aus der Klasse II auf ein und demselben Katalysatorkorn enthalten, vor allem den Vorteil der grösseren Lebensdauer und der Reproduzierbarkeit der Leistung.

Bei dem erfindungsgemässen Verfahren ist der Umsatz an δ-Ketocarbonsäureestern sowie die Ausbeute an Resorcinen relativ zur Edelmetallmenge wesentlich höher als bei den beiden genannten Gasphaseverfahren. Der Reaktionsdruck ist nicht kritisch; im allgemeinen arbeitet man bei Normaldruck, jedoch sind auch Über- oder Unterdruck geeignet. Ein besonders geeigneter Druckbereich ist 0.1 bis 10 bar. Die Reaktionstemperatur beträgt vorzugsweise 300 bis 400 °C.

Als Reste R im δ-Ketocarbonsäureester sind verzweigte oder geradkettige Alkylgruppen geeignet, beispielsweise Methyl, Ethyl, Propyl, Butyl, Pentyl, Hexyl, Octyl, Decyl und Dodecyl. Als Cycloalkylgruppen sind beispielsweise Cyclopentyl, Cyclohexyl, Cyclodecyl und Cyclododecyl geeignet. Bevorzugt sind Alkylgruppen oder Cycloalkylgruppen mit bis zu 6 Kohlenstoffatomen. Als Arylreste kommen die Phenyl- und die Naphtylgruppe in Frage. Vorteilhaft ist es, wenn mindestens zwei Reste R der Ausgangsverbindung Wasserstoff sind; besonders bevorzugt ist der Fall, dass alle Reste R = H sind.

Ist einer der Reste R eine Alkoxy- oder Carbalkoxygruppe, so ist es vorteilhaft, wenn die anderen Reste R = H sind. Der Alkylrest der Alkoxy- bzw. Carbalkoxygruppe ist vorzugsweise Methyl, Ethyl, n-Propyl oder n-Butyl.

Der Rest R' ist vorzugsweise eine geradkettige Alkylgruppe oder ein Arylrest wie Benzyl, Tolyl, Phenyl. Besonders bevorzugte Reste R' sind die Methyl-, Ethyl-, n-Propyl- und die n-Butylgruppe.

Als Ausgangsverbindung des erfindungsgemässen Verfahrens wird vorzugsweise der Methyl-, der Ethyl-, oder der n-Butylester der δ-Ketohexansäure oder die Methylester der folgenden Säuren: δ-Ketoheptansäure, γ-(n-Hexyl)-δ-Ketohexansäure, β-(n-Pentyl)-δ-Ketohexansäure, β-Carbomethoxy-δ-Ketohexansäure, γ-Methoxy-δ-Ketohexansäure und α-Methyl-β-Methyl-δ-Ketohexansäure verwendet.

Bevorzugte Elemente der Klasse I, die in Form von Verbindungen auf das Trägermaterial aufgebracht werden, sind Platin, Palladium, Iridium, Rhodium, Ruthenium und Osmium. Von diesen wiederum sind Palladium und Platin wegen ihrer hohen Leistung besonders bevorzugt, insbesondere aber Platin. Bezogen auf das Gesamtgewicht der ersten Katalysatorkomponente beträgt der Gewichtsanteil des Elements oder der Elemente der Klasse I - berechnet als Metall - 0.01 bis 10 Gew.-%, vorzugsweise 0.1 bis 3 Gew.-%.

Bevorzugte Elemente der Klasse II, die in Form von Verbindungen auf das Trägermaterial aufgebracht werden, sind Magnesium, Calcium, Strontium, Zinn, Blei, Zirkon, Scandium, Lanthan und die Lanthaniden, sowie Thorium und Uran. Von diesen wiederum werden Strontium, Zinn, Zirkon und Thorium wegen ihrer hohen Leistung besonders bevorzugt, insbesondere aber Thorium.

Bezogen auf das Gesamtgewicht der zweiten Katalysatorkomponente beträgt der Gewichtsanteil des Elementes oder der Elemente der Klasse II - berechnet als Metall - 0.05 bis 10 Gew.-%, vorzugsweise 0.5 bis 4 Gew.-%.

Bevorzugte Kombinationen von Elementen der Klasse I und Klasse II sind Palladium mit Zirkon, Platin mit Zirkon, Palladium und Platin mit Zinn, Palladium und Platin mit Strontium, Platin und Iridium mit Thorium, Palladium mit Thorium, Palladium und Platin mit Thorium bzw. Platin mit Thorium. Besonders bevorzugt werden die Kombinationen aus Platin mit Thorium, aus Palladium mit Thorium oder aus Palladium und Platin mit Thorium; insbesondere aber Platin mit Thorium.

Geeignete Trägermaterialien in den beiden Katalysatorkomponenten sind Kohle, Siliciumdioxid, Kieselsäure oder deren Gele, Tone, Silikate, Schamott, Tonerde, Chromoxid, Zinkoxid, Magnesiumoxid, Zirkondioxid, Borsäure, Aluminiumoxid sowie dessen Mischoxide, Alumosilikate, Spinelle und Zeolithe. Bevorzugt werden Kohle, Aluminiumoxid, Siliciumdioxid, sowie Aluminiumoxid - Chromoxid. Besonders bevorzugt ist Kohle.

Der Katalysator des erfindungsgemässen Verfahrens besteht aus 2 Komponenten, die im allgemeinen zu einem Gemenge zusammengebracht und so in ein Reaktionsrohr gefüllt werden, dass entweder eine einzige Zone oder mehrere voneinander getrennte Zonen entstehen. Die Verteilung der Teilchen der beiden Komponenten im Gemenge muss dabei nicht unbedingt gleichmässig sein; ebenso muss die Verteilung der Teilchen der beiden Komponenten im Reaktionsrohr nicht unbedingt gleichmässig oder regelmässig sein. Vorteilhafterweise wird jedoch ein Gemenge mit gleichmässiger Verteilung beider Komponenten verwendet und so in das Reaktionsrohr gebracht, dass das Gemenge vorzugsweise eine einzige Zone mit gleichmässiger Verteilung der Teilchen beider Komponenten bildet.

Das Volumenverhältnis der beiden Katalysatorkomponenten im Gemenge sollte 95:5 bis 5:95 sein, vorzugsweise 90:10 bis 10:90. Besonders bevorzugt wird ein Volumenverhältnis von 75:25 bis 25:75, insbesondere 50:50.

Katalysatoren mit besonders hoher Aktivität sind beispielsweise folgende Gemenge gleicher Volumenteile der Komponenten:

| 1. Komponente | 2. Komponente |
|---|---|
| Palladium und Platin/$Al_2O_3-Cr_2O_3$ | Strontium/Kohle |
| Platin und Iridium/$Al_2O_3$ | Thorium/$Al_2O_3$ |
| Palladium/$Al_2O_3-Cr_2O_3$ | Thorium/Kohle |
| Palladium und Platin/Kohle | Zirkon/Kohle |
| Palladium und Platin und Iridium/Kohle | Thorium/Kohle |
| Platin/Kohle | Thorium/Kohle |

Ein besonders bevorzugter Katalysator ist das Gemenge gleicher Volumenteile aus Platin/Kohle mit Thorium/Kohle.

Durch Verwendung des beschriebenen Katalysators gelingt es, δ-Ketocarbonsäureester mit hohem Umsatz und guter Selektivität in Resorcine bei gleichzeitig hoher Ausbeute relativ zur

Edelmetallmenge umzuwandeln. Beispielsweise kann Resorcin aus δ-Ketohexansäuremethylester mit einer Ausbeute von 80 bis 100 g Resorcin pro Gramm Edelmetall, einer Selektivität von 75 bis 85% und einem Umsatz von 85 bis 90% des Esters hergestellt werden.

Die Katalysatoren können, falls notwendig, mehrfach regeneriert werden. Dies geschieht im allgemeinen durch Überleiten eines Gasgemisches bestehend aus Sauerstoff und einem weiteren Gas, wie beispielsweise Stickstoff, Kohlendioxid, Wasserdampf oder einem Edelgas über den Katalysator im Reaktionsrohr. Die Temperatur im Katalysator wird dabei je nach Art des Trägermaterials bei 100 bis 500°C gehalten, vorzugsweise bei 250 bis 400°C. Der Sauerstoffgehalt des Gasgemisches beträgt im allgemeinen 0.5 bis 10 Vol.-%, vorzugsweise 1 bis 5 Vol.-%. Besonders geeignet ist ein Gemisch aus Luft und Stickstoff. Nach mehrstündiger Behandlung mit dem beschriebenen Gas wird der Katalysator z.B. bei Temperaturen von 100 bis 400°C mit einem Gemisch aus Wasserstoff und einem anderen Gas wie beispielsweise Stickstoff, Kohlendioxid, Wasserdampf oder einem Edelgas behandelt; danach hat er seine ursprüngliche Aktivität im wesentlichen wiedererlangt. Auch das Reaktionsgemisch $H_2$/Ketoester ist geeignet.

Die Herstellung der beiden Katalysatorkomponenten erfolgt durch Aufbringen mindestens einer Verbindung eines Elements der Klasse I bzw. Klasse II auf die entsprechenden Träger nach einem der üblichen Tränkverfahren. Werden mehrere Verbindungen von Elementen derselben Klasse auf ein und denselben Träger aufgebracht, so kann dies gleichzeitig oder nacheinander erfolgen. Schwerlösliche Verbindungen können in einem Überschuss an Lösungsmittel gelöst werden; das Trägermaterial wird dann in der Lösung suspendiert und das überschüssige Lösungsmittel wieder abgedampft, oder aber der Tränkvorgang wird mehrmals wiederholt. Weiterhin können in Wasser schwerlösliche Verbindungen durch Zusätze – wie beispielsweise Natriumchlorid zu Palladiumchlorid – in eine wasserlösliche Form gebracht werden. Solche Zusätze müssen nach dem Tränkvorgang nicht unbedingt entfernt werden. Geeignete Verbindungen der Elemente der Klasse I sind z.B. solche, die sich von Stickstoff enthaltenden Säuren ableiten, wie Nitrate, Nitrite, Cyanide und Rhodanide; weiterhin komplexe Verbindungen mit Sauerstoff enthaltenden Liganden wie Ammoniak oder Cyanid. Weiterhin eignen sich Sulfate, Chloride, Bromide oder halogenenthaltende Komplexverbindungen oder Carboxylate wie beispielsweise Acetate, Oxalate oder Tartrate oder von CH-aciden organischen Verbindungen (wie beispielsweise Acetylaceton) abgeleitete Verbindungen. Bevorzugte Verbindungen sind Palladiumdinitrat, Platindicyanid, Kaliumhexacyanoplatinat, Kaliumtetracyanoplatinat, Kaliumtetranitroplatinat, Tetramminpalladiumdichlorid, Palladiumdichlorid, Palladiumsulfat, Platintetrachlorid, Hexachlorplatinsäure, Kaliumhexachloroplatinat, Natriumtetrachloroplatinat, Kaliumhexabromoplatinat, Palladium- oder Platindiacetat, Palladium- oder Platinacetylacetonat. Besonders bevorzugt werden Hexachloroplatinsäure, Kaliumhexachloroplatinat, Kaliumtetracyanoplatinat, Palladiumdiacetat und Platindiacetat verwendet.

Geeignete Verbindungen der Elemente der Klasse II sind z.B. Nitrate, Nitrite, Halogenide, Sulfate, Carbonate, Acetate und Oxalate. Bevorzugt werden die Nitrate, Halogenide oder Oxalate verwendet.

Geeignete Lösungsmittel sowohl für die Verbindungen der Elemente der Klasse I als auch der Klasse II sind Wasser oder organische Lösungsmittel, wie beispielsweise Alkohole, Ketone, Nitrile, Carbonsäureester und Halogenkohlenwasserstoffe. Bevorzugt werden Methanol, Aceton, Methylenchlorid, Chloroform und deren Gemische, sowie Wasser oder Gemische von Wasser mit Aceton oder mit Methanol verwendet.

Die Durchführung des erfindungsgemässen Verfahrens erfolgt durch Überleiten der gasförmigen Ausgangsverbindung im Gemisch mit Wasserstoff über den Katalysator. Das molare Verhältnis Wasserstoff zu Ausgangsverbindung ist dabei im allgemeinen 1:1 bis 50:1, vorzugsweise 3:1 bis 15:1.

Ausser dem Wasserstoff können zusätzlich Gase wie beispielsweise Stickstoff, Edelgas, Kohlendioxid oder Wasserdampf anwesend sein. Als zusätzliches Gas wird bevorzugt Stickstoff verwendet, wobei man ein Gasgemisch der Zusammensetzung $H_2$:$N_2$= 4:1 bis 1:4 einsetzt.

Folgende Arbeitsweise hat sich zur Durchführung des erfindungsgemässen Verfahrens als besonders günstig erwiesen: Die Umsetzung des δ-Ketocarbonsäureesters erfolgt in einem senkrecht stehenden, elektrisch beheizten Reaktionsrohr; der Katalysator befindet sich im mittleren Teil des Reaktionsrohres. Die Ausgangsverbindung wird in einen Verdampfer gegeben, mit Wasserstoff oder einem Gemisch aus Wasserstoff und beispielsweise Stickstoff gemischt und über den Katalysator geleitet. Da der Druck für die Reaktion keine kritische Grösse ist, wird bei Normaldruck gearbeitet. Das Reaktionsgemisch wird am Ende des Reaktionsrohres kondensiert. Das gebildete Resorcin kann entweder durch Destillation oder durch Extraktion isoliert werden.

Vergleichsbeispiel 1

Es werden zwei Vergleichskatalysatoren A und B hergestellt, wobei gekörnte Kohle als Trägermaterial verwendet wird.

Der Katalysator A wird durch Imprägnieren mit einer wässrigen Lösung, die Hexachloroplatinsäure und durch Natriumchlorid gelöstes Palladiumdichlorid enthält, hergestellt; Katalysator B durch Imprägnieren mit einer wässrigen Lösung aus Hexachloroplatinsäure und durch Salzsäure gelöstes Palladiumdichlorid. Die Katalysatoren werden drei Stunden lang bei 100°C und 0.1 bar getrocknet.

Katalysator A enthält 2.4 Gew.-% Palladium, 0.6 Gew.-% Platin, 4 Gew.-% Natriumchlorid. Kataly-

sator B enthält 1.6 Gew.-% Palladium und 0.5 Gew.-% Platin.

Über jeweils 70 ml des Katalysators A bzw. B, die unmittelbar vor Gebrauch während 3 Stunden bei 320°C mit einem Gasgemisch aus 20 Nl $H_2$/h und 14 Nl $N_2$/h behandelt worden waren, wird in einem senkrecht stehenden, elektrisch beheizten Reaktionsrohr bei Temperaturen von 340 bis 345°C von oben her pro Stunde ein Gemisch aus 20 Nl Wasserstoff, 14 Nl Stickstoff und 3,2 Nl gasförmigem δ-Ketohexansäuremethylester geleitet. Das Reaktionsprodukt wird kondensiert und durch Gaschromatographie analysiert.

In Tabelle 1 sind die Umsätze an δ-Ketohexansäuremethylester und die Selektivitäten der Resorcinbildung während der Versuchsdauer von 75 Stunden angegeben.

Tabelle 1

| Versuchsdauer (Stunden) | 15 | | 30 | | 45 | | 60 | | 75 | |
|---|---|---|---|---|---|---|---|---|---|---|
| Katalysator | A | B | A | B | A | B | A | B | A | B |
| % Umsatz | 25 | 30 | 40 | 35 | 35 | 38 | 22 | 27 | 8 | 15 |
| % Selektivität (Resorcin) | 35 | 28 | 55 | 58 | 62 | 54 | 41 | 32 | 27 | 19 |

Vergleichsbeispiel 2

Es werden zwei Katalysatoren C und D hergestellt, die wie Katalysator A des Vergleichsbeispiels 1 beschaffen sind, aber zusätzlich 2 Gew.-% Thorium enthalten. Katalysator C wird hergestellt, indem zunächst Thorium als wässrige Thoriumnitratlösung auf gekörnte Kohle aufgebracht und nach Trocknung bei 100°C und 0.1 bar während 4 Stunden im Stickstoffstrom calciniert wird; danach wird Palladium und Platin wie beim Katalysator A aufgebracht. Zur Herstellung von Katalysator D wird der wie Katalysator A im Vergleichsbeispiel 1 hergestellte und während 3 Stunden bei 320°C mit 20 Nl $H_2$/h und 14 Nl $N_2$/h behandelte Katalysator mit einer wässrigen Thoriumnitratlösung getränkt und bei 100°C und 0.1 bar getrocknet.

Der Katalysator C bzw. D wird unter den Bedingungen des Vergleichsbeispiels 1, jedoch während einer längeren Versuchsdauer eingesetzt.

Tabelle 2 enthält die Umsätze an δ-Ketohexansäuremethylester und die Selektivitäten der Bildung von Resorcin während einer Versuchsdauer von 210 Stunden.

Tabelle 2

| Versuchsdauer (Stunden) | 30 | | 60 | | 90 | | 130 | | 170 | | 210 | |
|---|---|---|---|---|---|---|---|---|---|---|---|---|
| Katalysator | C | D | C | D | C | D | C | D | C | D | C | D |
| % Umsatz | 55 | 52 | 60 | 55 | 62 | 57 | 61 | 56 | 58 | 53 | 57 | 50 |
| % Selektivität (Resorcin) | 55 | 47 | 58 | 54 | 57 | 52 | 52 | 46 | 44 | 39 | 38 | 38 |

Dieses Beispiel zeigt, dass der Zusatz von Thorium den Umsatz der Reaktion und die Lebensdauer des Katalysators deutlich erhöht.

Beispiel 1

Es wird ein Katalysator E hergestellt, der aus zwei Katalysatorkomponenten besteht.

Die erste dieser Komponenten wird genauso hergestellt wie der Katalysator A aus Vergleichsbeispiel 1 und enthält 2.4 Gew.-% Palladium, 0.6 Gew.-% Platin und 4 Gew.-% Natriumchlorid. Die zweite Komponente enthält 2 Gew.-% Thorium und wird hergestellt durch Tränken von gekörnter Kohle mit einer wässrigen Lösung aus Thoriumnitrat, Trocknen bei 100°C im Vakuum und Calcinieren bei 400°C im Stickstoffstrom während 4 Stunden. Je 35 ml der beiden Komponenten werden innig vermengt und in das Reaktionsrohr gefüllt.

Der Katalysator E wird während 3 Stunden bei 330°C mit 30 Nl/h eines Gemisches aus Stickstoff:Wasserstoff = 2:3 behandelt. Danach werden bei 340 bis 345°C pro Stunde 20 Nl Wasserstoff, 14 Nl Stickstoff und 3.2 Nl gasförmiger δ-Ketohexansäuremethylester über den Katalysator geleitet.

In Tabelle 3 sind die Umsätze an δ-Ketohexansäuremethylester und die Selektivität der Resorcinbildung während der Versuchszeit von 480 Stunden zusammengefasst.

Tabelle 3

| Versuchsdauer (Stunden) | 48 | 72 | 144 | 288 | 384 | 480 |
|---|---|---|---|---|---|---|
| % Umsatz | 96 | 94 | 93 | 85 | 76 | 68 |
| % Selektiv. (Resorcin) | 66 | 68 | 72 | 71 | 68 | 64 |

Dieses Beispiel zeigt, dass durch Vermengung des Katalysators A mit einer Thorium enthaltenden weiteren Katalysatorkomponente der Umsatz, die Selektivität der Reaktion und die Lebensdauer des Katalysators deutlich erhöht wird.

Beispiel 2

Der in Beispiel 1 eingesetzte Katalysator wird nach 480 Stunden Versuchsdauer regeneriert. Dies geschieht durch Überleiten eines Gemisches aus 6 Nl Luft und 80 Nl Stickstoff pro Stunde über den Katalysator im Reaktor. Die Temperatur wird auf 350°C gehalten. Nach 6 Stunden werden während weiterer 3 Stunden 20 Nl Wasserstoff und 14 Nl Stickstoff pro Stunde über den Katalysator geleitet, dann die Reaktion wie in Beispiel 1 fortgeführt. Die Regeneration wird nach 720 Stunden Versuchsdauer wiederholt. Die Ergebnisse sind in Tabelle 4 zusammengefasst.

Tabelle 4

| Versuchsdauer (Stunden) | 480 | Regeneration | 576 | 672 | 720 | Regeneration | 816 | 912 | 960 |
|---|---|---|---|---|---|---|---|---|---|
| % Umsatz | 68 | | 95 | 85 | 72 | | 91 | 82 | 65 |
| % Selektivität (Resorcin) | 64 | | 65 | 68 | 59 | | 64 | 65 | 52 |

Beispiel 3

Es werden zwei Katalysatorkomponenten hergestellt, und zwar die erste Komponente X wie Katalysator A aus Vergleichsbeispiel 1, die zweite Komponente Y durch Tränken von gekörnter Kohle mit einer wässrigen Lösung von Thoriumoxalat:Ammoniumoxalat = 1:2.3, Trocknen und Calcinieren wie in Beispiel 1 beschrieben. Y enthält somit 2 Gew.-% Thorium auf Kohle, und X enthält 2.4% Pd, 0.6% Pt sowie 4% NaCL. Aus den Komponenten X, Y werden zwei Katalysatoren F und G wie folgt aufgebaut:

F: Man stellt aus 25 ml X und 50 ml Y fünf Gemenge zu je 15 ml her und zwar in den Proportionen

1. Y:X = 3 :3
2. Y:X = 3.5:2.5
3. Y:X = 4 :2
4. Y:X = 4.5:1.5
5. Y:X = 5 :1

Diese fünf Gemenge füllt man der Reihe nach in ein Reaktionsrohr, so dass fünf Zonen entstehen.

G: Man stellt aus 50 ml X und 25 ml Y fünf Gemenge zu je 15 ml her und zwar in den Proportionen

1. Y:X = 3 :3
2. Y:X = 2.5:3.5
3. Y:X = 2 :4
4. Y:X = 1.5:4.5
5. Y:X = 1 :5

Diese fünf Gemenge füllt man wieder der Reihe nach in ein Reaktionsrohr, so dass fünf Zonen entstehen.

Der Katalysator F bzw. G wird wie Katalysator E des Beispiels 1 eingesetzt. Tabelle 5 enthält die Ergebnisse.

Tabelle 5

| Versuchsdauer (Stunden) | 48 | | 72 | | 144 | | 216 | | 288 | |
|---|---|---|---|---|---|---|---|---|---|---|
| Katalysator | F | G | F | G | F | G | F | G | F | G |
| % Umsatz | 88 | 92 | 86 | 90 | 82 | 90 | 74 | 86 | 68 | 82 |
| % Selektivität (Resorcin) | 55 | 58 | 62 | 65 | 66 | 66 | 63 | 64 | 59 | 60 |

Dieses Beispiel zeigt, dass die Katalysatorkomponenten in verschiedenem Volumenverhältnis zueinander stehen können; der Katalysator kann aus mehreren Zonen unterschiedlicher Zusammensetzung bestehen.

Beispiel 4

Es werden 4 Katalysatorkomponenten analog zur Herstellung der zweiten Komponente des Beispiels 1 hergestellt, die aber statt Thorium jeweils 2 Gew.-% Calcium, Strontium, Zirkon oder Zinn auf gekörnter Kohle enthalten. 35 ml jeder Katalysatorkomponente werden mit jeweils 35 ml einer wie Katalysator A in Vergleichsbeispiel 1 hergestellten Komponente vermengt und wie Katalysator E (Beispiel 1), aber bei anderen Temperaturen zur Umsetzung von δ-Ketohexansäuremethylester herangezogen. Die erhaltenen Umsätze und Selektivitäten der Resorcinbildung sind in Tabelle 6 zusammengefasst.

Tabelle 6

| Versuchsdauer (Stunden) | | 72 | | 144 | |
|---|---|---|---|---|---|
| Element | Temperatur °C | % Umsatz | % Selektiv. (Resorcin) | % Umsatz | % Selektiv. (Resorcin) |
| Calcium | 320 | 82 | 56 | 71 | 52 |
| Strontium | 310 | 86 | 62 | 76 | 54 |
| Zirkon | 340 | 92 | 64 | 88 | 62 |
| Zinn | 350 | 86 | 58 | 78 | 55 |

Dieses Beispiel zeigt, dass die verwendeten Metalle ähnliche Wirkung wie Thorium haben, dass Thorium jedoch zu bevorzugen ist (vgl. Beispiel 1).

Beispiel 5

Es werden 4 Katalysatorkomponenten hergestellt, die jeweils 2 Gew.-% Thorium auf verschiedenen Trägern enthalten, und zwar durch Tränken von Aluminiumoxid (100 m²/g), Siliciumdioxid (120 m²/g), Silicagel (350 m²/g) bzw. Chromoxid-Aluminiumoxid (60 m²/g) mit wässriger Thoriumnitratlösung, Trocknen bei 100°C und 0.1 bar und anschliessendem Calcinieren bei 400°C während 4 Stunden. Je 35 ml jeder dieser Katalysatorkomponenten werden mit jeweils 35 ml frischem Katalysator A (s. Vergleichsbeispiel 1) vermengt und unter den Bedingungen von Beispiel 1 zur Umsetzung von δ-Ketohexansäuremethylester herangezogen. Die Umsätze und Selektivitäten der Resorcinbildung sind in Tabelle 7 zusammengefasst.

Tabelle 7

| Versuchsdauer (Stunden) | 72 | | 144 | |
|---|---|---|---|---|
| Träger | % Umsatz | % Selektiv. (Resorcin) | % Umsatz | % Selektiv. (Resorcin) |
| $Al_2O_3$ | 82 | 55 | 68 | 46 |
| $SiO_2$ | 80 | 58 | 62 | 52 |
| $SiO_2$-Gel | 88 | 61 | 79 | 55 |
| $Cr_2O_3$-$Al_2O_3$ | 86 | 56 | 72 | 48 |

Beispiel 6

Es werden 4 Katalysatorkomponenten hergestellt, die jeweils 1.2 Gew.-% Palladium und 0.3 Gew.-% Platin auf den Trägern Aluminiumoxid (100 m²/g), Siliciumdioxid (120 m²/g), Silicagel (350 m²/g) bzw. Chromoxid-Aluminiumoxid (60 m²/g) enthalten, und zwar durch Tränken der Trägermaterialien mit einer Chloroformlösung von Palladium- und Platinacetat und anschliessendes Trocknen bei 100°C.

Je 35 ml dieser 4 Katalysatorkomponenten werden jeweils mit 35 ml einer 2 Gew.-% Thorium auf Kohle enthaltenden zweiten Katalysatorkomponente vermengt und wie in Beispiel 1 beschrieben eingesetzt.

Umsätze und Selektivitäten der Resorcinbildung sind in Tabelle 8 zusammengefasst.

Tabelle 8

| Versuchsdauer (Stunden) | 72 | | 144 | |
|---|---|---|---|---|
| Träger | % Umsatz | % Selektiv. (Resorcin) | % Umsatz | % Selektiv. (Resorcin) |
| $Al_2O_3$ | 76 | 62 | 74 | 53 |
| $SiO_2$ | 68 | 58 | 58 | 43 |
| $SiO_2$-Gel | 66 | 52 | 62 | 46 |
| $Cr_2O_3$-$Al_2O_3$ | 96 | 83 | 92 | 79 |

Beispiel 7

Es werden 6 Katalysatoren durch Vermengen von jeweils gleichen Volumenteilen zweier Katalysatorkomponenten hergestellt. Der Tabelle 9 ist der Gewichtsanteil an Edelmetall (Iridium, Platin, Palladium) der Komponente I, sowie die Gewichtsanteile von Thorium bzw. Zirkon am Gewicht der Komponente II, weiterhin die jeweils verwendeten Trägermaterialien und die zur Herstellung der Komponenten eingesetzte Metallverbindung zu entnehmen. Die Herstellung jeder Katalysatorkomponente erfolgt mit Wasser als Lösungsmittel. Jede Komponente I bzw. II wird 3 Stunden lang bei 100°C und 0.1 bar getrocknet; jede der Komponenten II zusätzlich 4 Stunden lang bei 400°C im Stickstoffstrom calciniert. Die durch Vermengen der entsprechenden Komponenten I mit Komponenten II erhaltenen Katalysatoren werden zur Umsetzung von δ-Ketohexansäuremethylester unter Bedingungen des Beispiels 1 herangezogen.

Der Umsatz an δ-Ketohexansäuremethylester und die Selektivität der Resorcinbildung während 280 Stunden Versuchsdauer ist ebenfalls der Tabelle 9 zu entnehmen.

Tabelle 9

| Komponente I | | | Komponente II | | | | |
|---|---|---|---|---|---|---|---|
| % Metall | Trägermaterial | Metallverbindung zur Trägerimprägnierung | % Metall | Trägermaterial | Metallverbindung zur Trägerimprägnierung | Umsatz % | Selekt. % |
| 0.3 Ir | Kohle | $H_2IrCl_6$ | 1 Th | Kohle | $Th(NO_3)_4$ | 62 | 72 |
| 0.5 Pt | Kohle | $H_2PtCl_6$ | 2 Zr | $SiO_2$- | $Zr(NO_3)_4$ | 78 | 68 |
| 0.3 Ir | Kohle | $H_2IrCl_6$ | 2 Zr | Gel | | 78 | 68 |
| 0.3 Pt | Kohle | $K_2Pt(CN)_4$ | 1.5 Th | Kohle | $Th(NO_3)_4$ | 92 | 86 |
| 0.6 Pt | Kohle | $K_2PtCl_6$ | 2 Th | Kohle | $Th(NO_3)_4$ | 87 | 84 |
| 0.4 Pt | Kohle | $KCL + H_2PtCL_6$ | 2 Th | Kohle | $Th(NO_3)_4$ | 88 | 78 |
| 0.4 Pt | Kohle | $K_2PtCl_6$ | 1 Th | Kohle | $Th(Oxalat)_2$ | 77 | 74 |
| 0.7 Pd | Kohle | $NaCl + PdCl_2$ | 1 Th | Kohle | $Th(Oxalat)_2$ | 77 | 74 |

Beispiel 8

Es wird ein Katalysator hergestellt, der aus einem Gemenge gleicher Volumenteile einer Komponente I (0.3 Gew.-% Platin auf Chromoxid-Aluminiumoxid) und einer Komponente II (2 Gew.-% Thorium auf gekörnter Kohle) besteht. Platin wurde als Platinacetat in Chloroform, Thorium als Thoriumnitrat in Wasser aufgebracht.

70 ml dieses Katalysators werden nach dreistündigem Behandeln mit einem Gasgemisch aus 20 Nl Wasserstoff/h und 14 Nl Stickstoff/h bei 325°C zur Umsetzung von δ-Ketohexansäuremethylester bei 350°C eingesetzt. 20 g δ-Ketohexansäuremethylester werden stündlich verdampft, mit 30 Nl Wasserstoff und 20 Nl Stickstoff pro Stunde gemischt und über den Katalysator geleitet. Während 450 Stunden Versuchsdauer werden 8865 g Kondensat, aus welchem Resorcin auskristallisiert, erhalten. Die Analyse mittels Gaschromatographie ergibt folgende Zusammensetzung des Kondensats:

| | | | |
|---|---|---|---|
| Resorcin | 5265 g ≙ | 59.4 | Gew.-% |
| δ-Ketohexansäuremethylester | 900 g ≙ | 10.15 | Gew.-% |
| Methanol | 1557 g ≙ | 17.56 | Gew.-% |
| Phenol | 225 g ≙ | 2.53 | Gew.-% |
| Cyclohexanon | 153 g ≙ | 1.72 | Gew.-% |
| Hexansäuremethylester | 139 g ≙ | 1.57 | Gew.-% |
| Methylpropylketon | 122 g ≙ | 1.37 | Gew.-% |
| Cyclohexandion-1.3 | 85.5 g ≙ | 0.97 | Gew.-% |
| δ-Caprolacton | 86 g ≙ | 0.98 | Gew.-% |
| 3-Methoxy-cyclohexen-2-on | 67 g ≙ | 0.76 | Gew.-% |
| Resorcinmonomethylether | 58 g ≙ | 0.66 | Gew.-% |

Weiterhin sind 1.6 Gew.-% Wasser im Gemisch enthalten.

Die Bildung von Resorcin ist mit einer Selektivität von 85% bei einem Umsatz von 90% δ-Ketohexansäuremethylester verlaufen.

Der Katalysator wird, wie in Beispiel 2 beschrieben, regeneriert und die Reaktion weitere 300 Stunden lang fortgesetzt. Während dieser Zeit beträgt die Selektivität der Resorcinbildung 81%, der Umsatz von δ-Ketohexansäuremethylester 88%.

Nach wiederholtem Regenerieren und weiteren 300 Stunden Versuchsdauer beträgt die Selektivität 74%, der Umsatz 84%.

**Patentansprüche**

1. Verfahren zur Herstellung von Resorcinen der allgemeinen Formel

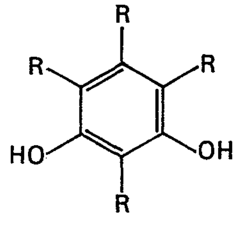

in der die einzelnen Reste R gleich oder verschieden und unabhängig voneinander Wasserstoff, Alkyl, Cycloalkyl, Aryl, Alkoxy oder Carbalkoxy

mit einer Gesamtzahl von bis zu 12 Kohlenstoffatomen sein können, durch Umsetzung von δ Ketocarbonsäureestern der allgemeinen Formel

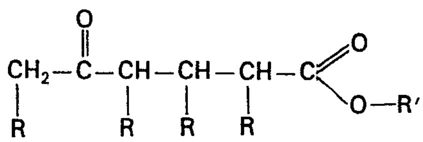

wobei R die obige Bedeutung hat und R' ein Alkyl-, Cycloalkyl- oder Aryl-Rest mit bis zu 8 Kohlenstoffatomen ist, in der Gasphase bei Temperaturen von 250 bis 500°C in Gegenwart von Wasserstoff an einem Katalysator, das dadurch gekennzeichnet ist, dass der Katalysator aus 2 Komponenten besteht, wobei die erste Komponente aus einem oder mehreren Trägermaterialien besteht, auf die jeweils mindestens eine Verbindung eines Metalls der Gruppe VIII und/oder mindestens eine Verbindung eines Metalls der Gruppe I B aufgebracht worden ist, und wobei die zweite Komponente aus einem oder mehreren Trägermaterialien besteht, auf die jeweils mindestens eine Verbindung eines Metalls der Gruppe II A und/oder IV A und/oder III B und/oder IV B des Periodensystems aufgebracht worden ist.

2. Verfahren nach Anspruch 1, dadurch gekennzeichnet, dass zur Herstellung der ersten Komponente mindestens eine Verbindung von Platin, Palladium, Iridium, Rhodium, Ruthenium oder Osmium verwendet wird, und dass der Gewichtsanteil dieses Elements am Gesamtgewicht der ersten Komponente - berechnet als Metall - 0.01 bis 10 Gew.-% beträgt.

3. Verfahren nach einem der Ansprüche 1 und 2, dadurch gekennzeichnet, dass zur Herstellung der ersten Komponente mindestens eine Verbindung von Platin oder Palladium verwendet wird.

4. Verfahren nach einem der Ansprüche 1 und 2, dadurch gekennzeichnet, dass zur Herstellung der ersten Komponente mindestens eine Verbindung von Platin verwendet wird.

5. Verfahren nach einem der Ansprüche 1 bis 4, dadurch gekennzeichnet, dass zur Herstellung der zweiten Komponente mindestens eine Verbindung von Magnesium, Calcium, Strontium, Zinn, Blei, Zirkon, Scandium, Lanthan, den Lanthaniden, Thorium oder Uran verwendet wird, und der Gewichtsanteil dieses Elements am Gesamtgewicht der zweiten Komponente - berechnet als Metall - 0.05 bis 10 Gew.-% beträgt.

6. Verfahren nach einem der Ansprüche 1 bis 5, dadurch gekennzeichnet, dass zur Herstellung der zweiten Komponente mindestens eine Verbindung von Strontium, Zinn, Zirkon oder Thorium verwendet wird.

7. Verfahren nach einem der Ansprüche 1 bis 5, dadurch gekennzeichnet, dass zur Herstellung der zweiten Komponente mindestens eine Verbindung von Thorium verwendet wird.

8. Verfahren nach einem der Ansprüche 1 bis 7, dadurch gekennzeichnet, dass der Katalysator ein Gemenge der ersten und der zweiten Komponente ist.

9. Verfahren nach Anspruch 8, dadurch gekennzeichnet, dass das Volumenverhältnis der beiden Komponenten 95:5 bis 5:95 beträgt.

10. Verfahren nach Anspruch 8, dadurch gekennzeichnet, dass das Volumenverhältnis der beiden Komponenten 90:10 bis 10:90 beträgt.

11. Verfahren nach Anspruch 8, dadurch gekennzeichnet, dass das Volumenverhältnis der beiden Komponenten 75:25 bis 25:75 beträgt.

12. Verfahren nach einem der Ansprüche 1 bis 11, dadurch gekennzeichnet, dass als Trägermaterialien Kohle, Aluminiumoxid, Siliciumdioxid oder Aluminiumoxid-Chromoxid verwendet wird.

13. Verfahren nach einem der Ansprüche 1 bis 12, dadurch gekennzeichnet, dass als Trägermaterial Kohle verwendet wird.

14. Verfahren zur Regenerierung eines Katalysators zur Herstellung von Resorcin aus δ -Ketocarbonsäureestern, der aus 2 Komponenten besteht, wobei die erste Komponente aus einem oder mehreren Trägermaterialien besteht, auf die jeweils mindestens eine Verbindung eines Metalls der Gruppe VIII und/oder mindestens eine Verbindung eines Metalls der Gruppe I B aufgebracht worden ist, und wobei die zweite Komponente aus einem oder mehreren Trägermaterialien besteht, auf die jeweils mindestens eine Verbindung eines Metalls der Gruppe II A und/oder IV A und/oder III B und/oder IV B des Periodensystems aufgebracht worden ist, dadurch gekennzeichnet, dass man über den Katalysator bei Temperaturen von 100 bis 500°C ein Gemisch von Sauerstoff mit Stickstoff, Kohlendioxid, Wasserdampf oder einem Edelgas leitet.

15. Verfahren nach Anspruch 14, dadurch gekennzeichnet, dass als Trägermaterial Kohle verwendet wird.

**Claims:**

1. Process for the manufacture of resorcinols of the formula

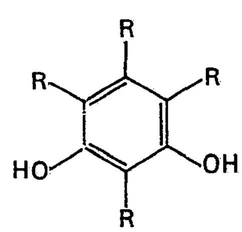

wherein the radicals R may be identical or different and, independent from one another, each are hydrogen, alkyl, cycloalkyl, aryl, alkoxy or Carbalkoxy having altogether up to 12 carbon atoms, by reaction of δ-ketocarboxylic acid esters of the formula

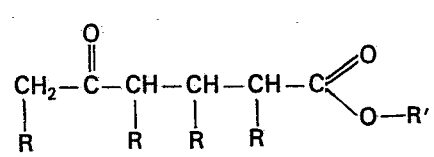

wherein R is defined as above and R' is alkyl, cycloalkyl or aryl having up to 8 carbon atoms, in the gaseous phase at a temperature of from 250 to 500°C in the presence of hydrogen at a catalyst, characterized in that the catalyst comprises two components, the first component consisting of one or more carrier materials, onto which at least one compound of a metal of group VIII and/or at least one compound of a metal of group I B has been applied and the second component consisting of one or more carrier materials, onto which at least one compound of a metal of group II A and/or IV A and/or III B and/or IV B of the periodic system has been applied.

2. Process as claimed in claim 1, characterized in that at least one compound of platinum, palladium, iridium, rhodium, ruthenium or osmium is used for the manufacture of the first component and wherein the proportion by weight of the corresponding element, relative to the total weight of the first component, calculated as metal, is in the range from 0.01 to 10 weight %.

3. Process as claimed in anyone of claims 1 and 2, characterized in that at least one compound of platinum or palladium is used for the manufacture of the first component.

4. Process as claimed in anyone of claims 1 and 2, characterized in that at least one compound of platinum is used for the manufacture of the first component.

5. Process as claimed in anyone of claims 1 to 4, characterized in that at least one compound of magnesium, calcium, strontium, tin, lead, zirconium, scandium, lanthanum, lanthanides, thorium or uranium is used for the manufacture of the second component and wherein the proportion by weight of the corresponding element, relative to the total weight of the second component, calculated as metal, is in the range from 0.05 to 10 weight %.

6. Process as claimed in anyone of claims 1 to 5, characterized in that at least one compound of strontium, tin, zirconium or thorium is used for the manufacture of the second compound.

7. Process as claimed in anyone of claims 1 to 5, characterized in that at least one compound of thorium is used for the manufacture of the second component.

8. Process as claimed in anyone of claims 1 to 7, characterized in that the catalyst used is a mixture of the first and second component.

9. Process as claimed in claim 8, characterized in that the volume ratio of both components is in the range of from 95:5 to 5:95.

10. Process as claimed in claim 8, characterized in that the volume ratio of the two components is in the range from 90:10 to 10:90.

11. Process as claimed in claim 8, characterized in that the volume ratio of the two components is in the range from 75:25 to 25:75.

12. Process as claimed in anyone of claims 1 to 11, characterized in that charcoal, aluminium oxide, silicon dioxide or aluminium oxide-chromium oxide are used as carrier materials.

13. Process as claimed in anyone of claim 1 to 12, characterized in that charcoal is used as carrier material.

14. Process for the regeneration of a catalyst for the manufacture of resorcinols from δ-keto-carboxylic acid esters, which catalyst comprises two components wherein the first component consists of one or more carrier materials, onto each of which at least one compound of a metal of group VIII and/or at least one compound of a metal of group I B have been applied and wherein the second component consists of one or more carrier materials, onto each of which at least one compound of a metal of group II A and/or IV A and/or III B and/or IV B of the periodic system have been applied, characterized by passing over the catalyst at a temperature from 100 to 500°C mixture of oxygen with nitrogen, carbon dioxide or a noble gas.

15. Process as claimed in claim 14, characterized by using as carrier material charcoal.

**Revendications**

1. Procédé de préparation de résorcinols répondant à la formule générale

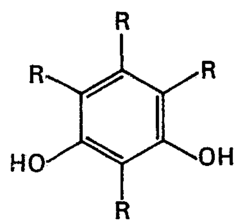

dans laquelle les divers symboles R sont identiques ou différents et représentent chacun, indépendamment les uns des autres un atome d'hydrogène ou un radical alkyle, cycloalkyle, aryle, alcoxy ou alcoxycarbonyle dont le nombre total d'atomes de carbone est d'au plus 12, par réaction d'esters d'acides δ-oxo-carboxyliques répondant à la formule générale

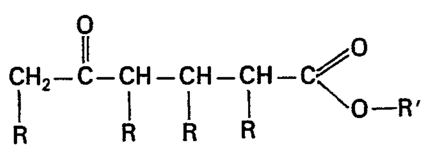

dans laquelle les R ont les significations précédemment données et R' représente un radical alkyle, cycloalkyle ou aryle contenant au plus 8 atomes de carbone, en phase gazeuse, à des températures de 250 à 500°C, en présence d'hydrogène et au contact d'un catalyseur, procédé caractérisé en ce que le catalyseur comprend deux composantes, la première étant constituée d'une ou plusieurs matières supports sur laquelle ou sur chacune desquelles a été déposé au moins un composé d'un métal du groupe VIII et/ou au moins un composé d'un métal du groupe I B, et la seconde étant constituée d'une ou plusieurs matières supports sur laquelle ou sur chacune desquelles a été déposé au moins un

composé d'un métal du groupe II A et/ou IV A et/ou III B et/ou IV B de la classification périodique.

2. Procédé selon la revendication 1, caractérisé en ce qu'on utilise, pour préparer la première composante, au moins un composé du platine, du palladium, de l'iridium, du rhodium, du ruthénium ou de l'osmium, et en ce que la proportion pondérale de cet élément par rapport au poids total de la première composante (calculée en métal) est comprise entre 0,01 et 10 % en poids.

3. Procédé selon l'une des revendications 1 et 2, caractérisé en ce que, pour préparer la première composante, on utilise au moins un composé du platine ou du palladium.

4. Procédé selon l'une des revendications 1 et 2, caractérisé en ce que, pour préparer la première composante, on utilise au moins un composé du platine.

5. Procédé selon l'une quelconque des revendications 1 à 4, caractérisé en ce que, pour préparer la seconde composante, on utilise au moins un composé du magnésium, du calcium, du strontium, de l'étain, du plomb, du zirconium, du scandium, du lanthane, des lanthanides, du thorium ou de l'uranium, et en ce que la proportion pondérale de cet élément par rapport au poids total de la seconde composante (calculée en métal) est comprise entre 0,05 et 10 % en poids.

6. Procédé selon l'une quelconque des revendications 1 à 5, caractérisé en ce que, pour préparer la seconde composante, on utilise au moins un composé du strontium, de l'étain, du zirconium ou du thorium.

7. Procédé selon l'une quelconque des revendications 1 à 5, caractérisé en ce que, pour préparer la seconde composante, on utilise au moins un composé du thorium.

8. Procédé selon l'une quelconque des revendications 1 à 7, caractérisé en ce que le catalyseur est un mélange de la première composante et de la seconde composante.

9. Procédé selon la revendication 8, caractérisé en ce que le rapport volumique des deux composantes est compris entre 95:5 et 5:95.

10. Procédé selon la revendication 8, caractérisé en ce que le rapport volumique des deux composantes est compris entre 90:10 et 10:90.

11. Procédé selon la revendication 8, caractérisé en ce que le rapport volumique des deux composantes est compris entre 75:25 et 25:75.

12. Procédé selon l'une quelconque des revendications 1 à 11, caractérisé en ce qu'on utilise, comme matières supports, le charbon, l'oxyde d'aluminium, le dioxyde de silicium ou l'oxyde mixte d'aluminium et de chrome.

13. Procédé selon l'une quelconque des revendications 1 à 12, caractérisé en ce qu'on utilise le charbon comme matière support.

14. Procédé pour régénérer un catalyseur qui est destiné à la préparation de résorcinols à partir d'esters d'acides δ-oxo-carboxyliques et qui comprend deux composantes, la première étant constituée d'une ou plusieurs matières supports sur laquelle ou sur chacune desquelles a été déposé au moins un composé d'un métal du groupe VIII et/ou au moins un composé d'un métal du groupe I B, et la seconde étant constituée d'une ou plusieurs matières supports sur laquelle ou sur chacune desquelles a été déposé au moins un composé d'un métal du groupe II A et/ou IV A et/ou III B et/ou IV B de la classification périodique, procédé caractérisé en ce qu'on fait passer sur le catalyseur, à des températures de 100 à 500°C, un mélange d'oxygène avec de l'azote, du dioxyde de carbone, de la vapeur d'eau ou un gaz rare.

15. Procédé selon la revendication 14, caractérisé en ce qu'on utilise le charbon comme matière support.